# EUROPÄISCHE PATENTSCHRIFT

(11) **EP 0 659 350 B1**
(45) Veröffentlichungstag und Bekanntmachung des Hinweises auf die Patenterteilung: **20.12.2000**
(21) Anmeldenummer: 94115928.7
(22) Anmeldetag: 10.10.1994
(51) Int. Cl.: A23L 2/46, A23L 2/54, C02F 1/72

(54) **Verfahren zum Geniessbar- und/oder Haltbarmachen von Wasser**
Process for making water palatable and/or for preserving it
Méthode pour rendre de l'eau comestible ou/et pour la conserver

(30) Priorität: 19.11.1993 DE 4339494
(43) Veröffentlichungstag der Anmeldung: 28.06.1995
(73) Patentinhaber: tegut...Gutberlet Stiftung & Co., D-36039 Fulda (DE)
(72) Erfinder: Gutberlet, Wolfgang, D-36160 Dipperz (DE); Schlinzig, Eckhart, Dr., D-53783 Eitorf (DE)
(74) Vertreter: Schlagwein, Udo, Dipl.-Ing.

(56) Entgegenhaltungen:
- EP-A- 0 469 211
- WO-A-88/06411
- DE-A- 2 926 441
- DE-A- 3 228 959
- DATABASE WPI Week 9240, Derwent Publications Ltd., London, GB; AN 92-327612 & JP-A-4 234 967 (ASAHI BREWERIES LTD.) 24. August 1992
- PATENT ABSTRACTS OF JAPAN vol. 16, no. 001 (C-899) 7. Januar 1992 & JP-A-03 228 645 (MITSUI PETROCHEM. IND. LTD.) 9. Oktober 1991
- DATABASE WPI Week 8910, Derwent Publications Ltd., London, GB; AN 89-073869 & JP-A-1 027 458 (CUBIC ENGINEERING K) 30. Januar 1989

## Beschreibung

Die Erfindung betrifft ein Verfahren zum Genießbar- und/oder Haltbarmachen von Wasser durch Erhitzen des Wassers, anschließendes Abkühlen und Behandeln in einem Behälter mit Sauerstoff.

Ein Verfahren der vorstehenden Art ist Gegenstand der EP-A-0 469 211. Bei dem in dieser Schrift beschriebenen Verfahren wird der Sauerstoff unter Druck in einen geschlossenen Behälter eingebracht.

Nicht abgekochtes Wasser enthält häufig Keime, welche nach einiger Zeit die Beschaffenheit des Wassers derart verändern, dass es ungenießbar werden oder sich geschmacklich nachteilig verändern. Oftmals muss Wasser abgekocht oder erhitzt werden, damit es überhaupt ohne gesundheitliche Gefährdung getrunken oder in Nahrungsmittel verarbeitet werden kann.

Durch das Abkochen oder Erhitzen von Wasser verändert sich sein Geschmack nachteilig, was leicht feststellbar ist, wenn man den Geschmack frischen Quellwassers mit dem von abgekochtem Wasser der gleichen Quelle vergleicht. Ähnliche Effekte sind bei anderen Flüssigkeiten, beispielsweise Obstsäften, zu beobachten.

Zur Vermeidung solcher nachteiligen Effekte durch Erhitzen oder Abkochen verzichtet man oftmals auf ein Erhitzen und fügt stattdessen ein keimtötendes Mittel bei, beispielsweise Chlor in Wasser. Solche Mittel beeinflussen jedoch ebenfalls den Geschmack des Wassers. Weiterhin können sie beim Genuss des Wassers zu Nebenwirkungen, insbesondere Allergien, führen. Da solche Nebenwirkungen kaum zu erforschen und selten ihre Ursache sicher feststellbar ist, bemüht man sich heute, mit möglichst wenig Konservierungsstoffen auszukommen.

Der Erfindung liegt das Problem zugrunde, ein Verfahren zum Genießbar- und/oder Haltbarmachen von Wasser zu entwickeln, welches den Geschmack des Wassers möglichst wenig verändert und zu keinen Nebenwirkungen auf Lebewesen durch Genuss dieses Wassers führt.

Dieses Problem wird erfindungsgemäß dadurch gelöst, dass der Behälter als Schüttelbehälter ausgebildet ist, zur Behandlung der Flüssigkeit mit Sauerstoff oberhalb ihres Wasserspiegels Luft enthält und zur Erhöhung des Sauerstoffgehaltes des Wassers der Behälter etwa 2,5 Minuten geschüttelt wird.

Die DE-A-42 15 637.8 beschreibt zwar schon ein Verfahren zur Verbesserung der Haltbarkeit von Flüssigkeiten, bei dem die Flüssigkeiten zwei bis drei Minuten lang in einem Behälter geschüttelt werden. Die Anwendung dieses Verfahrens bei abgekochten oder hocherhitzten Flüssigkeiten erscheint jedoch abwegig, weil abgekochte Flüssigkeiten ohnehin keimfrei sind und deshalb nicht haltbarer gemacht werden müssen.

Durch dieses erfindungsgemäße Schütteln nach dem Erhitzen und Abkühlen des Wassers gelangt der Sauerstoff, welcher beim Erhitzen aus dem Wasser ausgetrieben wurde, erneut in das Wasser hinein. Dadurch wird die durch das Erhitzen auftretende geschmackliche Einbuße des Wassers wieder rückgängig gemacht und das Wasser durch den Sauerstoff aktiviert. Die Erfindung erlaubt es deshalb, das für die Vernichtung von Keimen besonders zuverlässige und wirkungsvolle Verfahren des Erhitzens des Wassers anzuwenden, ohne dass die dabei bislang zu beobachtenden geschmacklichen Nachteile eintreten. Abgesehen vom besseren Geschmack ist Wasser mit höherem Sauerstoffgehalt auf den Körperorganismus wirksamer, da der in dem Wasser vorhandene Sauerstoff beim Verdauungsvorgang direkt in die Blutbahn gelangt. Das Schütteln kann in einem offenen Behälter in Luftatmosphäre, aber auch in einem geschlossenen Behälter erfolgen.

Als besonders vorteilhaft hat sich erwiesen, wenn das Wasser auf eine Temperatur von mindestens 80° erhitzt und vor dem Schütteln bis in die Nähe der Raumtemperatur abgekühlt wird. Messungen mit Leitungswasser und mit Brunnenwasser zeigten, dass durch Erhitzen auf 80°C der Sauerstoffgehalt von etwa 9,5 mg/l auf etwa 6,6 mg/l sank, nach dem erfindungsgemäßen Schütteln jedoch wieder auf den ursprünglichen Wert anstieg. Dieser durch das Schütteln angehobene Sauerstoffgehalt bleibt im Wasser über Wochen erhalten, wenn man es in geschlossenen Behältern aufbewahrt.

## Patentansprüche

1. Verfahren zum Genießbar- und/oder Haltbarmachen von Wasser durch Erhitzen des Wassers, anschließendes Abkühlen und Behandeln in einem Behälter mit Sauerstoff, **dadurch gekennzeichnet**, dass der Behälter als Schüttelbehälter ausgebildet ist, zur Behandlung des Wassers mit Sauerstoff oberhalb des Flüssigkeitsspiegels Luft enthält und zur Erhöhung des Sauerstoffgehaltes des Wassers der Behälter etwa 2,5 Minuten geschüttelt wird.

2. Verfahren nach Anspruch 1, **dadurch gekennzeichnet**, dass das Wasser auf eine Temperatur von mindestens 80° erhitzt und vor dem Schütteln bis in die Nähe der Raumtemperatur abgekühlt wird.

## Claims

1. Process for making water palatable and/or capable of preservation by heating the water and then cooling it and treating it with oxygen in a container, characterized in that the container is formed as a shaking container, contains air above the surface of the liquid for treatment of the water with oxygen, and is shaken for about 2½ minutes to raise the oxygen content of the water.

2. Process according to Claim 1, characterized in that the water is heated to a temperature of at least 80° and is cooled to around room temperature before shaking.

## Revendications

1. Procédé pour rendre l'eau potable et/ou apte à la conservation par chauffage de l'eau, refroidissement ultérieur et traitement dans un récipient avec de l'oxygène, caractérisé en ce que le récipient constitué par un récipient à secousses que, pour le traitement du liquide par l'oxygène, il contient de l'air au-dessus de la surface libre du liquide et que, pour accroître la teneur de l'eau en oxygène, le récipient est secoué pendant environ 2,5 minutes.

2. Procédé selon la revendication 1, caractérisé en ce qu'on chauffe l'eau à une température d'au moins 80°C et qu'avant le secouage, on la refroidit jusqu'au voisinage de la température ambiante.
